# EUROPEAN PATENT APPLICATION

(11) **EP 1 666 885 A2**
(43) Date of publication of application: **07.06.2006**
(21) Application number: 05386025.0
(22) Date of filing: 05.12.2005
(51) Int. Cl.: G01N 33/68

(54) **Proteins with prognostic, diagnostic and therapeutic importance in trisomy 21 (Down syndrome)**

(30) Priority: 06.12.2004 GR 2004100471
(71) Applicant: Foundation of Biomedical Research of the Academy of Athens, 115 27 Athens (GR); Tsangaris, George, 152 35 Vrilissia Attikis (GR); Karamessinis, Panagiotis, 185 37 Pireus (GR); Kolialexi, Aggeliki, 115 21 Athens (GR); Garbis, Spididon, 115 27 Athens (GR); Fountoulakis, Michail, 115 27 Athens (GR); Mavrou-Kalpini, Ariadni, 145 78 Ekali Attikis (GR); Antsaklis, Aristidis, 115 28 Athens (GR)
(72) Inventor: Tsangaris George, 152 35 Vrilissia Attikis (GR); Kolialexi, Aggeliki, 115 21 Athens (GR); Karamessinis, Panagiotis, 185 37 Pireus (GR); Garbis, Spiridon, 115 27 Athens (GR); Fountoulakis, Michail, 115 27 Athens (GR); Mavrou-Kalpini, Ariadni, 148 78 Ekali Attikis (GR); Antsaklis, Aristidis, 115 28 Athens (GR)
(74) Representative: Tefou-Fotea, Eleni

(57) **Abstract**

We report on the identification of seven (7) proteins showing quantitative and/or qualitative differences in amniotic fluid samples obtained from women carrying fetuses with Trisomy 21 (Down syndrome) as compared to those obtained from women carrying chromosomally normal ones. Proteins identified include CA11 (P02452), CA13 (P02461), CA15 (P20908), PGBM (P98160), SFRS4 (Q08170), AMBP (P02760), and IBP1 (P08833). These proteins, as whole molecules or parts of their amino acid sequence, represent as a total or each one separately or in different combinations, targets and can be used as markers for prognosis and/or diagnosis of Trisomy 21. Additionally, these proteins or their fragments, as a total or each one of them or in combinations could be therapeutic targets and could be used for the treatment of Trisomy 21 in utero or postnatally.

## Description

### B. FIELD OF THE INVENTION

The present invention refers to seven (7) proteins showing quantitative and qualitative differences in amniotic fluid samples obtained from pregnancies carrying fetuses with Down syndrome as compared to those with chromosomally normal fetuses. The presence of these proteins as whole molecules or fragments of their sequence can represent markers for prognosis, treatment or prenatal (invasive or non-invasive) detection of Down syndrome. All 7 proteins can be used in total or each one separately or even in different combinations.

### C. BACKGROUND OF THE INVENTION

Today the methodology used for the prenatal diagnosis of fetal chromosomal abnormalities includes invasive and non-invasive techniques. Invasive procedures (amniocentesis, trophoblast biopsy and fetal blood) carry a significant risk for the mother and the fetus and for this reason they are recommended only in pregnant women in whom the risk to obtain a chromosomally abnormal child is higher than the risk of fetal loss due to the invasive technique.

Since the risk for chromosomal abnormalities increases with maternal age, prenatal diagnosis is recommended for women over 35 years of age. As a result, fewer children with Down syndrome are born from older women. However, 70% of the children with this chromosomal abnormality are born to women 35 years of age for whose prenatal diagnosis is not recommended.

Non invasive prenatal diagnosis methods routinely used today for chromosomal abnormalities are recommended to low risk pregnancies. These methods include both biochemical and ultrasonographic markers which after algorithmic estimation are used only to evaluate the risk for chromosomal abnormalities. In cases with abnormal biochemical or ultrasonographic markers further investigation is recommended by invasive prenatal diagnosis. Additionally these markers are not specific for Trisomy 21 and are associated with a 5% false positive detection rate, leading to an increase in the number of women undergoing prenatal diagnosis.

New non-invasive techniques for prenatal diagnosis based on the isolation of fetal cells or cell free fetal DNA from maternal peripheral blood seem to be difficult to be applied into clinical practice due to lack of the specific characteristics to distinguish between fetal and maternal material.

Nowadays, proteomic analysis is widely used for the detection of biological markers related to diseases. This technology can use all types of biological fluids and tissues as well as peripheral blood, leading to the development of fast, accurate and low cost clinical tests which can dramatically alter current diagnostic procedures and therapeutic approaches.

A limited number of published data on proteomic analysis of amniotic fluid and peripheral blood of pregnant women are related to the detection of proteins and peptides during gestation as a result of the molecular disturbances in DNA or RNA level. These studies show that proteomic analysis is a versatile approach for the detection of markers related to pathological conditions during pregnancy and these proteins/peptides can be used as markers of these conditions.

Studies of the brain from fetuses and adults with DOWN syndrome showed disturbances of enzymes participating in the metabolism of glucose, lipids, purins, folic acid and methionine, decreased N-methyltransferase of histamine, and disturbances in the expression of PRXIII of the brain, implying that more than one gene encodes for proteins related to the phenotype of the syndrome.

In the present invention we detected and identified proteins, which whole or their fragments from the sequence, can be targets and be used originally as markers for the invasive or non-invasive prenatal diagnosis of Down syndrome from all the women independently of risk for having a chromosomally abnormal fetus as well as for the treatment of Down syndrome during pregnancy or postnatally.

### D. DESCRIPTION OF THE INVENTION

Five amniotic fluid samples obtained from pregnant women carrying fetuses with Trisomy 21 (Down syndrome) and 10 from cases carrying chromosomally normal ones were analyzed using proteomic techniques as described in details below.

### Methods

Proteomic analysis includes proteins separation by two dimensional gel electrophoresis and protein identification by mass spectrometry. Protein identification procedure, apart from analytical techniques, includes specific software which is used to analyze two dimensional gels and to store obtained information into data bases.

### A. Protein separation by two dimensional gel electrophoresis.

The technique is based on the simultaneous separation of proteins by their charge and molecular weight resulting in the isolation within the gel of hundreds of proteins. Thus, such a study includes the protein separation by their charge (isoelectic focusing, IEF) followed by further separation based on their molecular weight.

The IEF is an equilibration procedure by which proteins move by the action of a high voltage electric field, across an acrylamide gel with differential pH, containing immobilines (IPG strips overlaid). During their movement across the strip proteins stabilize and focuse on areas of the strip in which their charge is neutralized. The IPG stips are available in a great range of pH (such as 3-10) and size (7-24cm), as well as to more closed pH ranges (such as 4.5-5.5). IPG strips have the advantage to analyze a relatively high amount of sample. Elecrophoresis conditions for IEF are related to the amount of the sample, the method used for the application of the sample on the strip, voltage and time of focusing.

At the second dimension, protein separation is based on the molecular weight. It takes place on constant or gradient polyacrylamide gel depending on the approximate size of proteins. Commonly constant 12% acrylamide and gradient 9-16% acrylamide gels are used for protein separation between 5-200kDa. Finally gels are stained with dyes. Coumassie blue is usually selected when proteins have to be further identified by mass spectrometry.

Given the heterogeneity of proteins, each protein can be represented on the two dimensional gel by more than one spot, resulting in the identification of a higher number of gene products (proteins) than this of coded genes. In eukaryotics, it is known that 5-20 different proteins can be translated by the same gene.

### B. Mass spectrometry.

Following separation by two dimensional electrophoresis, proteins are identified either by 1) Mass Spectrometry (Matrix-assisted laser desorp tion- ionization, MALDI-ToF-MS) or 2) Ion spray techniques Protein spots are excised from acrylamide gel, distained and treated with trypsin. All deriving peptides, from all spots are collected and analyzed by mass spectrometry and amino acid sequence determination of each peptide. Sophisticated software are used to compare the masses of detected peptides to the theoretical masses of all available protein data sets of all species. Protein identification is normally based on the detection of more than five peptides matching for each protein (up to twenty peptides are sometimes matched in cases of large protein molecules). Thus false detection rate is estimated to be less than 10⁻⁶.

Ion spray technique may provide safe results in amino acid sequencing of peptide with a probability of false rate less than 10⁻¹⁰, related to the number of peptides used for the analysis.

In conclusion, MALDI-TOF-MS technique allows rapid analysis of a large number of protein spots. However, ion spray technique is more reliable for protein identification. Those two techniques are usually used simultaneously and in tight correlation with each other in order to identify significant differences in protein expression between health and disease state and to determine their use as biological markers of therapeutic targets.

All technologies mentioned above, were used to analyze 5 amniotic fluid samples obtained from pregnancies carrying fetuses with Down syndrome (IMAGE 2) and 10 from cases carrying chromosomally normal ones (IMAGE 1). When obtained proteomic profiles were compared 7 proteins CA11, CA13, CA15, PGBM, AMBP, IBP1, SFRS4. (IMAGES 3 and 4 and TABLE I) were found to be differentially expressed in cases with Trisomy 21.

These proteins, as whole molecules or fragments, represent biological markers to be used for prognosis, prenatal diagnosis as well as therapeutic targets for Trisomy 21 (Down syndrome).

**TABLE I: PROTEINS DIFFERENTIALLY EXPRESSED IN CASES CARRYING FETUSES WITH DOWN SYNDROME**

| **No** | **Symbol** | **Accession Number** | **Mass (Daltons)** | **Complete Name** |
|---|---|---|---|---|
| 1 | CA11 | P02452 | 139825 | Collagen alpha 1 (I) chain precursor |
| 2 | CA13 | P02461 | 139724 | Collagen alpha 1 (III) chain precursor |
| 3 | CA15 | P20908 | 184189 | Collagen alpha 1 (V) chain precursor |
| 4 | PGBM | P98160 | 479248 | Basement membrane-specific heparan sulfate proteoglycan core protein precursor (HSPG) (Perlecan) (PLC) |
| 5 | AMBP | P02760 | 39886 | AMBP protein precursor Contains: Alpha -1-microglobulin (protein HC) (Complex-forming glycoprotein heterogeneous in charge) (Alpha-1 microglycoprotein) |
| 6 | IBP1 | P08833 | 28912 | Insulin-like growth factor binding protein 1 precursor (IGFBP-1) (IBP-1) (IGF-binding protein 1) (Placental protein 12) (PP12) |
| 7 | SFRS4 | Q08170 | 56759 | Splicing factor, arginine/serine-rich 4 (pre-mRNA splicing factor SRP75) (SRP001 LB) |

### E. ADVANTAGES OF THE CURRENT INVENTION

The methodology currently used for the prenatal diagnosis of fetal chromosomal abnormalities includes invasive and non-invasive techniques. Invasive procedures (amniocentesis, trophoblast biopsy and fetal blood) carry a significant risk for the mother and the fetus and for this reason they are recommended only in pregnant women in whom the risk to obtain a chromosomally abnormal child is higher than the risk of fetal loss due to the invasive technique.

Since the risk for chromosomal abnormalities increases with maternal age, prenatal diagnosis is recommended for women over 35 years of age. As a result, fewer children with Down syndrome are born from older women. However, 70% of the children with this chromosomal abnormality are born to women 35 years of age for whose prenatal diagnosis is not recommended.

Non invasive prenatal diagnosis methods routinely used today for chromosomal abnormalities are recommended to low risk pregnancies. These methods include both biochemical and ultrasonographic markers which after algorithmic estimation are used only to evaluate the risk for chromosomal abnormalities. In cases with abnormal biochemical or ultrasonographic markers further investigation is recommended by invasive prenatal diagnosis. Additionally these markers are not specific for Trisomy 21 and are associated with a 5% false positive detection rate, leading to an increase in the number of women undergoing prenatal diagnosis.

New non-invasive techniques for prenatal diagnosis based on the isolation of fetal cells or cell free fetal DNA from maternal peripheral blood seem to be difficult to be applied into clinical practice due to lack of the specific characteristics to distinguish between fetal and maternal material.

In the current invention, we report on 7 proteins, CA11, CA13, CA15, PGBM, AMBP, IBP1 and SFRS4 showing qualitative or quantitative differences in amniotic fluid samples obtained from cases with Trisomy 21 compared to the normal ones. These proteins as whole molecules or their fragments, can be targets and be used originally as markers for invasive or non-invasive prenatal diagnosis of DOWN syndrome from all women independently of the risk for having a chromosomally abnormal fetus as well as for the treatment of DOWN syndrome in utero or postnatally.

### F. IMAGE DESCRIPTION

IMAGE 1. Representative image of two dimensional polyacrylamide gel of amniotic fluid obtained from gestation of normal embryo, produced by the application of the relevant methodologies. Each spot on the gel represents one protein.

IMAGE 2. Representative image of two dimensional polyacrylamide gel of amniotic fluid obtained from gestation with Trisomy 21 embryo (Down Syndrome). Gel is produced by the application of the relevant methodologies and each spot represents one protein.

IMAGE 3. Comparison of two dimensional polyacrylamide gel of amniotic fluid obtained from gestation of a chromosomal normal embryo (left image) and of two dimensional polyacrylamide gel obtained from a gestation of a Trisomy 21 pathological embryo (image on the right). Arrows indicate spots and mentioned proteins (CA15 and PGBM) which appear to be different amongst the two gel images. Differences which appear in squares A and B furthermore indicate important regions and are analyzed in detail on image 4.

IMAGE 4. Comparison and analysis of regions A and B of the polyacrylamide gels from Image 3 (in a 2X magnification). Region A is part of polyacrylamide gel image of amniotic fluid obtained from gestation of chromosomally normal embryo, whilst Region B represents part of polyacrylamide gel image of amniotic fluid obtained from gestation of embryo with Trisomy 21 (Down syndrome). Arrows indicate proteins mentioned before (CA11, CA13, AMBP, SFRS4 and IBP1), which appear to be different amongst the two gel images. In Region B, proteins CA13 and

CA11 appear to be enhanced (up regulated) in comparison to Region A, whilst protein SFRS4 is represented only by one spot, in Region B. Protein IBP1 in Region B is represented only by one spot and protein AMBP is represented by 8 spots, whilst in Region A IBP1 protein is represented by five spots and AMBP by three spots.

### G. DETAILED DESCRIPTION OF AN APPLICATION

Amniotic fluid obtained from women from 16^{th} up to 18^{th} week of gestation is centrifuged in 3000 rpm for 15min in order to precipitate all cellular components and to obtain supernatant for further analysis. The sample's supernatant can also be stored in - 80°C until further process. Next, measurement of the protein concentration of the sample follows using Bradford assay and through nanocapillary electrophoresis in nano-cartridges using Bioanalyzer 2100 (Agilent Tech).

Sample quantity consisting at least 2mg of total protein is mixed with 3 times volume of acetone and is stored in -20°C for 16-18 hours. The mixture is then centrifuged at 5000rpm for 15min in 4°C. The supernatant discard and the pellet re-dilute in 500µl sample buffer (consisting of: 20 mM Tris, 7 M Urea, 2 M Thiurea, 4% CHAPS, 10 mM 1,4 Dithrioethytol, 1 mM EDTA, 1 mM PMSF, protease inhibitors, 0,2 mM Na₂VO₃ and 1 mM NaF). Protein concentration is determined and 1 mg of total protein is analyzed by the methodology described in detail above. First step of the analysis is about protein separation according to their electric charge (isoelectric focusing, IEF) in a 3-10pH strip (1^{st} dimension). Focusing starts at 250V for 30 min and rises linearly up to 6000V during the next 14 hours and stays stable at 6000V for another 18hours in the appropriate device. After IEF, proteins are separated according to their molecular weight (2^{nd} dimension) by application of the strip to a 12% polycrylamide gel and a 6hour electrophoresis at 15 Watt/gel. After electrophoresis, gels are fixated in a solution of methanol (50%) and phosphoric acid (5%). Gels are then stained in a colloidal coomassie solution for about 16hours. In the case in which the embryo has Trisomy 21, spots of Table1 appear on the final gel in a different color volume, shape and number compared to the case in which the embryo is normal. Those spots are excised from the gel and are then being identified with mass spectrometry as mentioned above.

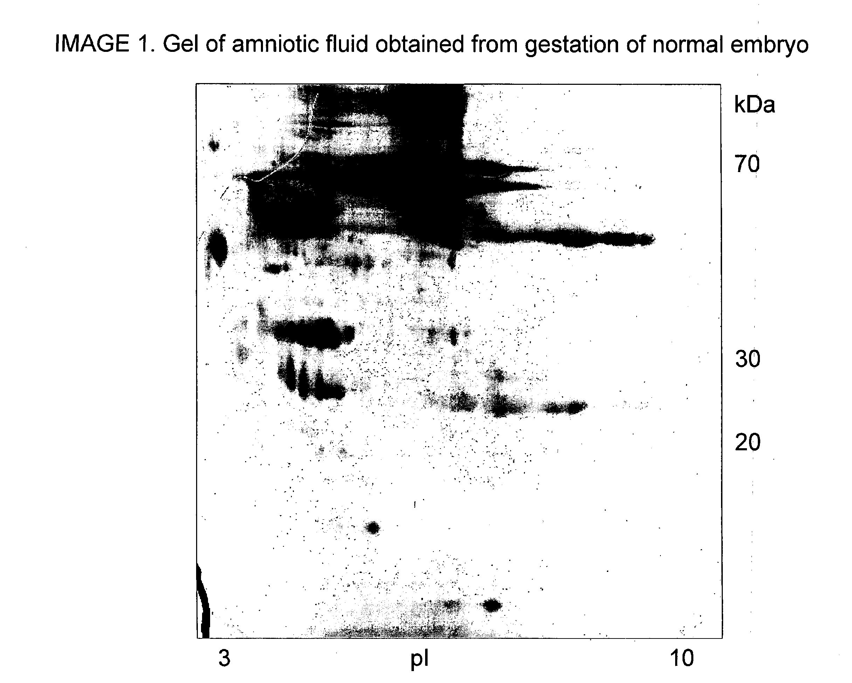

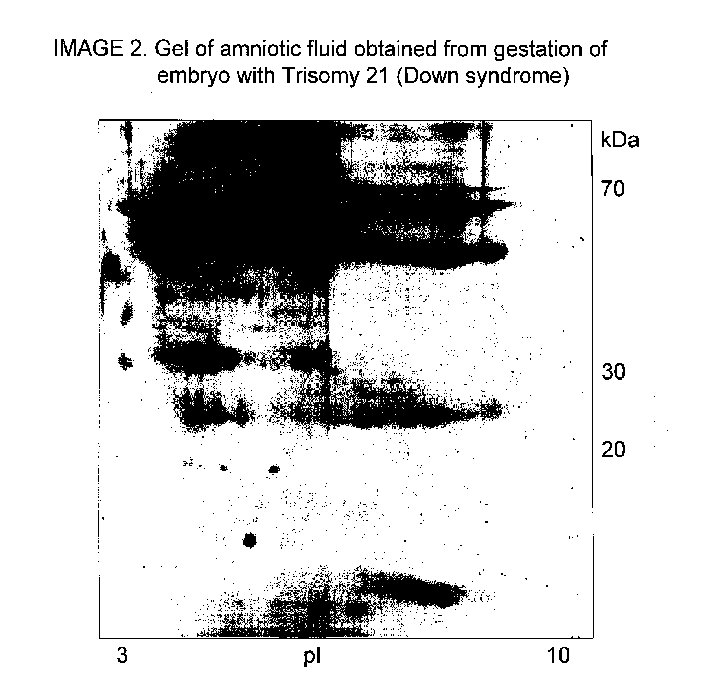

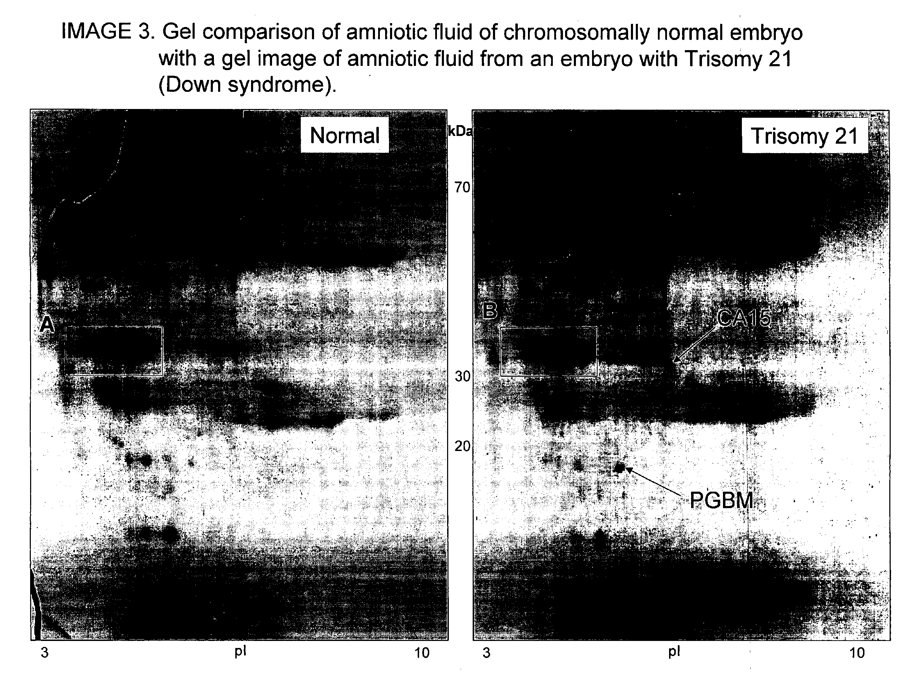

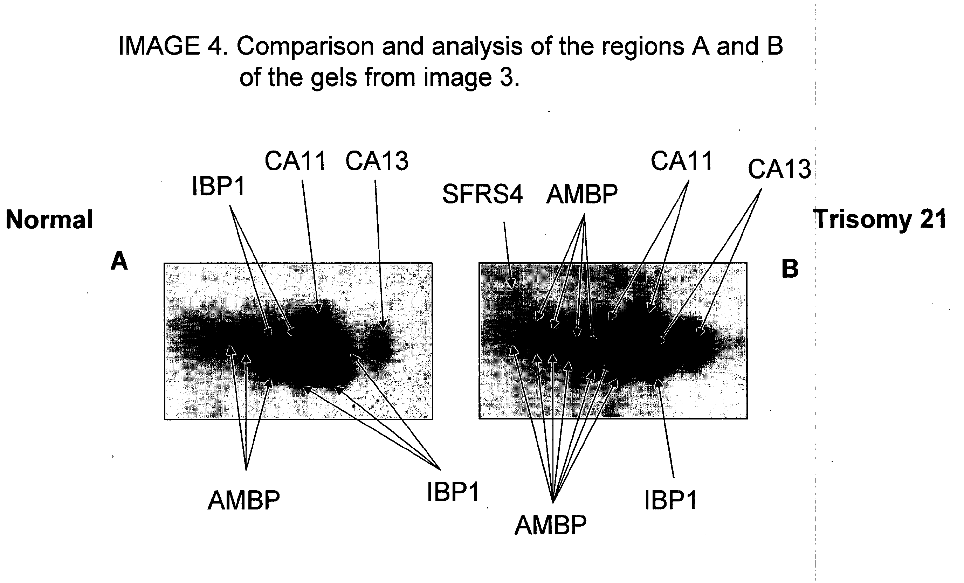

## Claims

1. A method for prognosis and/or diagnosis of Trisomy 21 (Down syndrome) in fetuses including:
a. sampling of biological fluid from a pregnant woman and
b. qualitative and quantitative detection of one or more proteins of the protein group: CA11 (P02452), CA13 (P02461), CA15 (P20908), PGBM (P98160), SFRS4 (Q08170), AMBP (P02760), and IBP1 (P08833) or protein fragments of their amino acid sequence, **characterized by** the comparison of values which were obtained in stage (b) with reference values for the same biological fluid in women carrying chromosomically normal fetuses.

2. A method which, according to claim 1, is **characterized by** the fact that the result for the prognosis and/or diagnosis is positive if the quantity of one or more of the proteins CA11 (P02452), CA13 (P02461), CA15 (P20908), PGBM (P98160), SFRS4 (Q08170), AMBP (P02760), and IBP1 (P08833) or protein fragments of their amino acid sequence is higher than the quantity mentioned as normal.

3. A method which, according to claim 1, is **characterized by** the fact that the result for the prognosis and/or diagnosis is positive if the quantity of protein AMBP (P02760) or fragment of it's amino acid sequence is higher than the quantity mentioned as normal.

4. A method which, according to claim 1, is **characterized by** the fact that the result for the prognosis and/or diagnosis is positive if the quantity of protein IBP1 (P08833) or part of it's amino acid sequence is lower than the quantity mentioned as normal.

5. A method which, according to claims 1 to 4, is **characterized by** the fact that the result for prognosis and/or diagnosis is positive if the ratio of the amount of the protein AMBP (P02760) or a part of it's amino acid sequence to the amount of the protein IBP1 (P08833) or a part of it's amino acid sequence, is different than the normal one.

6. A method which, according to claims 1 to 5, is **characterized by** the fact that the result for prognosis and/or diagnosis is positive if the ratio of the amount of one or more of the proteins CA11 (P02452), CA13 (P02461), CA15 (P20908), PGBM (P98160), SFRS4 (Q08170) or a parts of them, to the amount of the protein IBP1 (P08833) or a part of it's amino acid sequence, is different than the normal one.

7. A method which, according to each of the claims 1 to 6, is **characterized by** the fact that the biological fluid is peripheral blood, plasma, serum, urines, or amniotic fluid.

8. A method which, according to each of the claims 1 to 7, is **characterized by** the fact that the qualitative and/or quantitative analysis is performed using one of the following methods: nanotechnology, nanocapilary electrophoresis in a microchip, electrophoresis (of one or two dimensions), microtitration (simple and/or multiple ELISA), chromatography (HPLC, LC, nanoLC, GC etc), mass spectrometry [peptide mass fingerprinting, post source decay, MALDI MS, MALDI MS-MS, LC-MS, LC-MS-MS, TANDEM-MS, TANDEM MS-MS etc] or combination of these techniques.

9. A method which, according to each of the claims 1 to 8, is **characterized by** the fact that qualitative and/or quantitative analysis is performed using fluorescent substances or isotopes or antibodies or oligonucleotides or combination of these substances.

10. A method which, according to claims 1 to 9, is **characterized by** the fact that prognosis and/or diagnosis is considered as positive if the pattern of the contribution of the proteins or their fragments, in the area of 25-40 kDa molecular weight is different than that considered as normal.

11. A method which, according to claim 10, is **characterized by** the fact that the prognosis and/or diagnosis is considered as positive if the pattern of contribution of proteins with an intermediate molecular weight or fragments of these proteins in the molecular weight area of 25-40 kDa is different than the one considered as normal.

12. The use of one or more of the following proteins CA11 (P02452), CA13 (P02461), CA15 (P20908), PGBM (P98160), SFRS4 (Q08170), AMBP (P02760), and IBP1 (P08833) or fragments of their amino acid sequence, or factors inhibiting their activity and/or cleavage and/or their expression of the above proteins, for the production of a pharmaceutical product to be used for prognosis, diagnosis, precaution and/or therapeutic treatment of Trisomy 21 (Down syndrome).

13. The use, according to claim 12, of one or more factors inhibiting the cleavage of one or more of the following proteins CA11 (P02452), CA13 (P02461), CA15 (P20908), PGBM (P98160) and SFRS4 (Q08170).

14. The use, according to claim 12, of one or more factors which inhibit the cleavage and/or decrease the quantity and/or decrease the activity of the protein AMBP (P02760) or fragments of the sequence.

15. The use, according to claim 12 of one or more factors which increase the quantity and/or the activity of protein IBP1 (P08833).

16. A package (kit) including reagents and media for prognosis and/or diagnosis of the Trisomy 21 (Down syndrome) **characterized by** the fact that it includes factors for the identification and/or conjunction of one or more proteins of the following group: CA11 (P02452), CA13 (P02461), CA15 (P20908), PBGM (P98160), SFRS4 (Q08170), AMBP (P02760) and IBP1 (P08833) or fragments of their amino acid sequence.

17. A package (kit), according to the claim 16, including appropriate reagents and media for the application of one of the following methods: nanotechnology, nanocapillary electrophoresis in microchip, microtitration (simple or multiple ELISA), electrophoresis (one or two dimension), chromatography (HPLC, LC, nanoLC, GC etc), mass spectrometry (peptide mass fingerprint, post source decay, MALDI-MS, MALDI-MS-MS, LC-MS, LC-MS-MS, TANDEM-MS, TANDEM-MS-MS etc) or combination of these techniques.

18. A package (kit), according to the claims 16 and 17, including fluorescent dyes or isotopes or antibodies or oligonucleotides or combination of the above.
